# EUROPEAN PATENT SPECIFICATION

(11) **EP 2 686 422 B1**
(45) Date of publication and mention of the grant of the patent: **07.06.2017**
(21) Application number: 12757040.6
(22) Date of filing: 13.03.2012
(51) Int. Cl.: C12N 5/22, G01N 33/50, B82Y 30/00, C12N 5/077, B82Y 5/00

(54) **CARBON-NANOTUBE MODULATION OF CARDIAC MYOCYTE CELLS**
KOHLENSTOFFNANORÖHRCHEN-MODULATION VON HERTZMUSKELZELLEN
MODULATION DE MYOCYTES CARDIAQUES À L'AIDE DE NANOTUBES DE CARBONE

(30) Priority: 14.03.2011 US 201161452574 P
(43) Date of publication of application: 22.01.2014
(73) Proprietor: The Regents of the University of Colorado, a body corporate, Denver, CO 80203 (US); I.C.G.E.B. International Centre for Genetic Engineering and Biotechnology, 34149 Trieste (IT); University of Trieste, 34127 Trieste (IT)
(72) Inventor: MESTRONI, Luisa, Boulder CO 80305 (US); BALLERINI, Laura, I-34124 Trieste (IT); LONG, Carlin, Denver CO 80220 (US); CALDWELL, John, Denver CO 80231 (US); PRATO, Maurizio, Trieste (IT); MARTINELLI, Valentina, Turriaco Go (IT); CELLOT, Giada, Trieste (IT); TOMA, Francesca, Maria, I-30038 Spinea (IT); ZENTILIN, Lorena, I-34136 Trieste (IT)
(74) Representative: Moore, Barry
(86) International application number: PCT/US2012/028930
(87) International publication number: WO 2012/125638

(56) References cited:
- WO-A1-2010/060080
- US-A1- 2008 076 816
- US-A1- 2010 104 652
- US-A1- 2010 183 698
- VALENTINA MARTINELLI ET AL: "Carbon Nanotubes Promote Growth and Spontaneous Electrical Activity in Cultured Cardiac Myocytes", NANO LETTERS, vol. 12, no. 4, 20 March 2012 (2012-03-20) , pages 1831-1838, XP055174499, ISSN: 1530-6984, DOI: 10.1021/nl204064s
- VIVIANA LOVAT ET AL: "Carbon Nanotube Substrates Boost Neuronal Electrical Signaling", NANO LETTERS, vol. 5, no. 6, 1 June 2005 (2005-06-01), pages 1107-1110, XP055174621, ISSN: 1530-6984, DOI: 10.1021/nl050637m
- MACDONALD ET AL.: 'Collagen?carbon nanotube composite materials as scaffolds in tissue engineering.' JOURNAL OF BIOMEDICAL MATERIALS RESEARCH vol. 74A, no. 3, 22 June 2005, pages 489 - 496, XP002448802 Retrieved from the Internet: <URL:http://onüneübrary.wiley.com/doil10.10 02fjbm.a.303861pdf> [retrieved on 2012-06-07]
- SILVANO GARIBALDI ET AL: "Carbon nanotube biocompatibility with cardiac muscle cells", NANOTECHNOLOGY, IOP, BRISTOL, GB, vol. 17, no. 2, 28 January 2006 (2006-01-28), pages 391-397, XP020104373, ISSN: 0957-4484, DOI: 10.1088/0957-4484/17/2/008
- ANONYMOUS: 'ECACC General Cell Collection: 88092904 H9c2 (2-1)', [Online] 01 January 1976, XP055316995 Retrieved from the Internet: <URL:http://www.phe-culturecollections.org. uk/products/celllines/generalcell/detail.js p?refId=88092904&collection=ecacc_gc> [retrieved on 2016-11-07]
- A F BRANCO ET AL: 'Gene Expression Profiling of H9c2 Myoblast Differentiation towards a Cardiac-Like Phenotype' PLOS ONE, [Online] 29 June 2015, SAN FRANCISCO CA US, XP055316992 Retrieved from the Internet: <URL:https://www.ncbi.nlm.nih.gov/pmc/artic les/PMC4485408/> [retrieved on 2016-11-07]

## Description

### FIELD OF THE INVENTION

The present disclosure relates generally to the field of carbon nanotube compositions, and in particular, to carbon nanotube - primary cardiac tissue selected cardiac myocyte complexes and methods of making and using the same.

### BACKGROUND

Myocyte cells are highly specialized, terminally differentiated cells found in muscle tissue, including smooth muscle, skeletal muscle and cardiac tissue. Chronic and acute conditions and disease in such tissue are often caused by damage or improper function of the myocyte cells.

Irregular myocyte function has been implicated in various diseases and conditions, including atherosclerosis, peripheral vascular disease, dyslipidemia, hyperbetalipoproteinemia, hypoalphalipoproteinemia, hypercholesterolemia, hypertriglyceridemia, familial-hypercholesterolemia, cardiovascular disorders, angina, ischemia, cardiac ischemia, stroke, myocardial infarction, reperfusion injury, angioplastic restenosis, cardiomyopathy, heart failure, hypertension, vascular complications from diabetes, bladder disease, and skeletal muscle disease including muscular dystrophies, and related conditions among others.

Carbon nanotubes (CNTs) have been at the forefront of nanotechnology due to their unique features, which allow the development of a variety of miniaturized devices with remarkable properties. However, the ability of CNTs to modulate certain biological systems, particularly myocyte cells, is largely unknown.

The prior art includes an article written by VALENTINA MARTINELLI ET AL, titled "Carbon Nanotubes Promote Growth and Spontaneous Electrical Activity in Cultured Cardiac Myocytes" published in NANO LETTERS, vol. 12, no. 4 on 20 March 2012 (2012-03-20), pages 1831-1838 describing the demonstration that neonatal rat ventricular myocytes cultured on substrates of multiwall carbon nanotubes interacted with carbon nanotubes by forming tight contacts and showed increased viability and proliferation.

The prior art also includes an article written by VIVIANA LOVAT ET AL titled "Carbon Nanotube Substrates Boost Neuronal Electrical Signaling", published in NANO LETTERS, vol. 5, no. 6 on 1 June 2005 (2005-06-01), pages 1107-1110, which describes the possibility of using carbon nanotubes (CNTs) as potential devices for improving neural signal transfer while supporting dendrite elongation and cell adhesion.

The article by MACDONALS ET AL, titled "Collagen-carbon nanotube composite materials as scaffolds in tissue engineering" published in the JOURNAL OF BIOMEDICAL METERIALS RESEARCH, vol. 74A, no. 3 on 22 June 2005, describes the use of collagen-CNT composite matrices as scaffolds in tissue engineering, or as components of biosensors or other medical devices.

The state of the art is also indicted by the following patent publications: WO2010060080, US2010104652, US2010183698 and US2008076816.

### SUMMARY OF THE INVENTION

The present invention relates generally to carbon nanotube (CNT) compositions that are complexed with primary cardiac myocyte selected from cardiac tissue and methods of making and using the same according to the appended claims.

In various aspects, CNT complexes with primary cardiac myocyte cells selected from cardiac tissue are provided. In further aspects, methods of improving the electrophysical properties of said cardiac myocyte cells are provided. In still further aspects, methods of stimulating the proliferative capacity of said cardiac myocyte cells are provided.

Further aspects include the carbon nanotube cardiac myocyte complexes of the present invention for use in methods of treating subjects, wherein such subjects suffer, or are predisposed to suffer from, arrhythmia, conduction disease, cardiomyopathy, heart failure, atherosclerosis, peripheral vascular disease, dyslipidemia, hyperbetalipoproteinemia, hypoalphalipoproteinemia, hypercholesterolemia, hypertriglyceridemia, familial-hypercholesterolemia, cardiovascular disorders, angina, ischemia, cardiac ischemia, stroke, myocardial infarction, reperfusion injury, angioplastic restenosis, hypertension, and vascular complications of diabetes; bladder disease and conditions; and skeletal muscle disease, including muscular dystrophies.

### BRIEF DESCRIPTION OF THE DRAWINGS

FIG. **1A** shows a line graph of thermogravimetric analysis (TGA) curves; FIG. **1B** shows Sagittal TEM images of a CNT layer; FIG. **1C** and FIG. **1D** show TEM images of cardiomyocyte and CNT interaction; and FIG. **1E** and FIG. **1F** show SEM micrographs of a cardiac cell on CNT substrate.

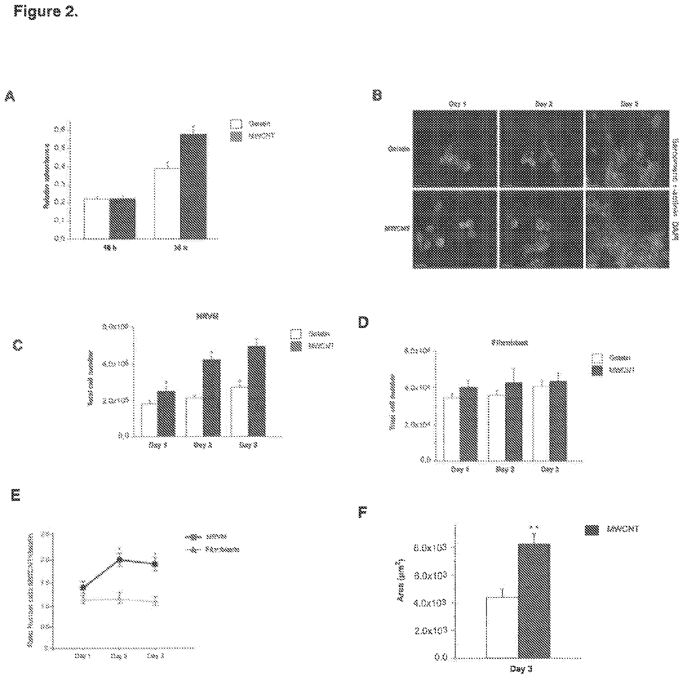

FIG. **2A** shows a graph of an AlmarBlue Viability Assay as indicator of cardiomyocyte proliferation and metabolism; FIG. **2B** shows representative images of purified cardiomyocytes stained for α-actinin at different time points in culture; FIG. **2C** shows a graph of total neonatal rat ventricular myocytes (NRVMs) on gelatin and CNT substrates at different time points in culture; FIG. **2D** shows a graph of total fibroblasts cells from newborn rats on gelatin and CNT substrates at different time points in culture; FIG. **2E** shows a graph of the ratio of total number of NRVMs and fibroblasts growing on carbon nanotubes and gelatin at the different time points; FIG. **2F** shows a graph of size of the syncytia beating domains NRVMs in gelatin control cultures and in carbon nanotube cultures, both measured at day 3. Note that syncytia are significantly increased when NRVM are grown on MWCNT substrates (P= 0.018).

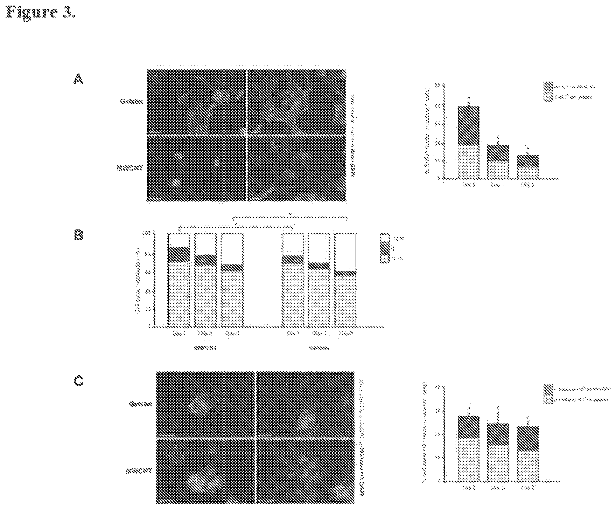

FIG. **3A****, left panel,** shows representative images of immunofluorescence for BrdU and α-actinin on cardiomyocytes plated on gelatin (top)- and CNT (bottom)- substrates at day 2 of culture. Nuclei were stained with DAPI; FIG 3A, right panel, shows quantification of BrdU-positive myocardial cells at different times in culture, FIG. **3B****, left panel** shows a graph of BrdU-positive myocardial cells at different times in culture; FIG. **3C****, left panel,** shows representative images of positivity to phosphorilated-histone-pH3 (Ser28) immunostaning on gelatin (top)-, and CNT (bottom)- substrates, nuclei stained with DAPI; and FIG. **3C****, right panel,** shows a graph of quantification of P-histone H3 (Ser28)- positive nuclei of myocardial cells at day 1, bars at 50 µm.

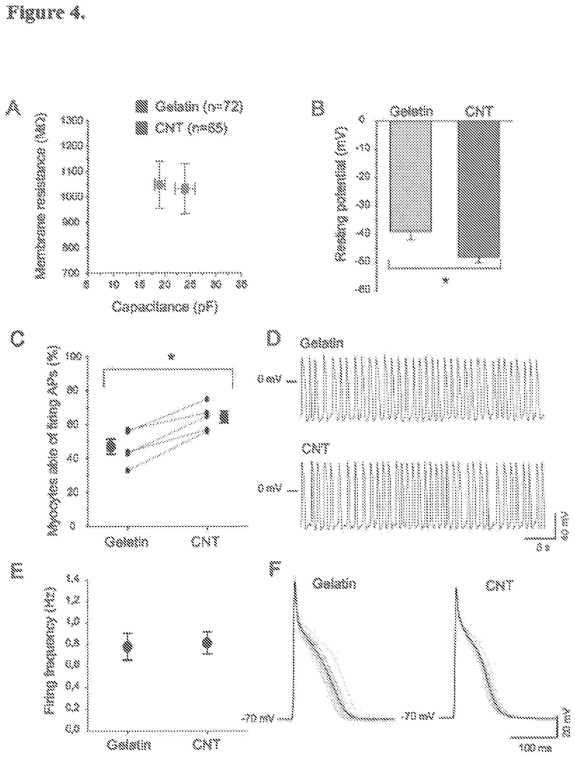

FIG. **4A** shows a graph of capacitance versus input resistance for CNT and gelatin cultures; FIG. **4B** shows a graph of resting potential for cardiomyocytes grown in CNT versus a control; FIG. **4C** shows a graph of myocytes ability to fire action potentials (APs) in the presence of CNTs versus a control; FIG. **4D** shows a chart of depicting sample recordings of spontaneous activity from myocytes on CNT substrates and a control; FIG. **4E** is a graph depicting the frequency of firing between CNT and a control; and FIG. **4F** is a graph depicting the measured kinetic parameters of evoked APs.

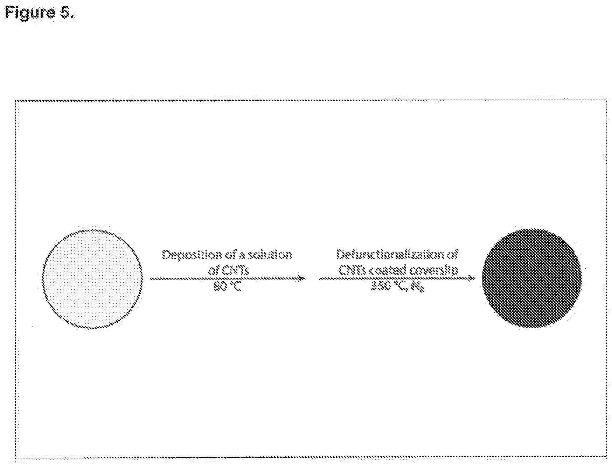

FIG. **5** shows a schematic representation of multi-wall carbon nanotube deposition over glass substrate and subsequent defunctionalization.
FIG. **6A** shows a scanning electron microscopy image of a multi-wall carbon nanotube; and FIG. **6B** shows a scanning electron microscopy image of an amorphous carbon substrate. The calibration bar is at 1 µm.
FIG. 7 shows a graph depicting proliferation of α actinin-positive cells grown on gelatin, multi-wall carbon nanotube cultures (CNT), amorphous carbon (AC), and Indium-Tin-Oxide glass (ITOglass), monitored at day 1, 2 and 3.
FIG. **8** shows a graph depicting C2C12 myoblast proliferation on Gelatin and MWCNT substrates, monitored at day 1 and 2.
FIG. **9A** shows a representative image of C2C12 myoblast stained for actin cytoskeleton and Propidium Ioide for nuclei (20X magnification). FIG. **9B** shows a representative image of C2C12 myoblasts with bi-nucleated C2C12 pseudo-myotube (60X magnification). On carbon nanotubes, the skeletal myocytes show a more organized structure with some multinucleated fibers, and pseudo-myotubes; both are signs of active proliferation and fusion of myoblasts. Bars: 50 µm.

### DETAILED DESCRIPTION OF THE INVENTION

Except to the extent defined otherwise, all technical and scientific terms used herein have the same meaning as commonly understood by one of ordinary skill in the art.

A "nanotube" is defined herein as a cylindrical nanostructure, having a diameter less than about 200 nanometers (nm). In certain embodiments, the dimensions of a nanotube comprises a length at least about ten times its diameters. In certain other embodiments, the dimensions of a nanotube are optionally and optimally defined as having a length from about 10 microns (µm) to about 10 nanometers, and a diameter from about 0.5 nm to about 100 nm.

A "carbon nanotube" is a crystalline carbon structure in which a thin layer of graphite crystal is rolled up into a cylindrical shape. CNTs may be formed from carbon atoms in the form of a graphene structure, which is a flat or curved layer formed by arranging six-membered rings of carbon atoms in a honeycomb arrangement.

A "plurality of carbon nanotubes" is defined herein as one or more discrete carbon nanotube particles or structures.

The term "complex," "complexe," "carbon nanotube complexe," and the like, is defined herein as a nanostructure that includes a carbon nanotube bound to, adjoined, or otherwise is in chemical, structural, and/or electrical communication with an atom, molecule, or structure, e.g., a myocyte cell. Such binding may be covalent or non-covalent or mixtures thereof. For example, carbon nanotubes may be complexed with a cellular surface of a myocyte cell or extra-cellular matrix associated with a myocyte cell. In various aspects and embodiments, the complex includes functional groups. The functional groups may be complexed for any suitable purpose, including, among others, facilitating additional modes of binding to a target myocyte cell.

A "dispersing agent" is defined herein as any suitable agent capable of dispersing a plurality of carbon nanotubes. Dispersing agents include any suitable inorganic or organic solvent, including ethanol, saline, and N,N'-dimethylformammide (DMF).

In accordance with various aspects, a CNT composition is provided. In various aspects and embodiments, CNTs are complexed with one or more myocyte cells. The myocyte cells are selected from cardiac tissue, smooth muscle tissue, and skeletal muscle tissue. Preferably, the myocyte cells are selected from cardiac tissue,

CNTs may be provided in any suitable form. CNTs may be selected from single-walled carbon nanotubes (SWCNTs), double-walled carbon nanotubes (DWCNTs) or multi-walled carbon nanotubes (MWCNTs), or a plurality containing combinations thereof. SWCNTs may be formed by a single graphene layer rolled up in a cylindrical form. Various structural shapes or patterns are provided, including "armchair," "chiral," and a "zig-zag" configuration of six-membered rings. DWCNTs and MWCNTs may be formed by two or more graphene layers rolled up in a cylindrical form. In certain preferred embodiments, CNTs may comprise MWCNTs.

CNTs may be formed using any suitable method, including arc-discharge methods, laser evaporation methods, chemical vapor deposition methods, and flowing vapor deposition methods. The arc-discharging method involves growing CNTs by arc discharge using carbon electrodes. The laser evaporation method involves evaporating part of a graphite electrode by means of a laser. Chemical vapor deposition is typically a thermal process involving thermally decomposing hydrocarbon on a substrate with a metal catalyst. The flowing vapor deposition method involves flowing an organic transition metal compound and a hydrocarbon compound in a carrier gas, and cross-reacting at high temperature.

In accordance with various aspects, CNTs are deposited onto a substrate, such as, for example, a glass substrate. Deposition onto the substrate may proceed according to any suitable method. In various aspects, the CNT is suspended in a dispersant solution. The solution is deposited onto the substrate. Deposition may proceed and/or repeat until achieving a desired volume and/or thickness. In various embodiments, the desired volume is from about 0,02 ml to about 20 ml, and in a further embodiment about 2ml. The desired density may be from about 7x10⁻⁴ mg/mm² to about 7x10⁻⁶ mg/mm², and in a further embodiment about 7x10⁻⁵ mg/mm².

CNT compositions may be functionalized prior to complexing with a myocyte cell. CNTs have a high capacity for adsorption due to their large surface area. The surface area of CNTs may be further adjusted by altering the associated surface chemistry. CNTs may be chemically treated, for example, with an acid treatment, to render the surface more accessible for adsorption of certain compounds, for example, a protein. Acid treatment may induce defects on the protein surface as well as promote de-bundling, which can be correlated with an increase in surface area, Nitric acid treatment may introduce carboxylic acid groups at sites of defects, thereby increasing the capacity of protein adsorption. CNTs may also be reduced following acid treatment. For example, following nitric acid treatment, CNTs may be treated with lithium borohydride to reduce oxygenated groups created after acid treatment. Such treatment may favor dispersion of CNTs in solution. In various aspects, the CNT compositions are functionalized prior to complexing with a myocyte cell.

In addition to non-specific adsorption, proteins can be covalently attached to CNTs though various functional groups. The term "functionality" or "functionalized" refers to conjugation of a molecule to the surface of a CNT via a functional chemical group (e.g., carboxylic acids, aldehydes, amines, sulfhydryl and hydroxyl groups, among others) present on the CNT and/or the molecule to be attached. For example, CNTs can be functionalized by attaching chemical compounds and other complexing units for use as vectors for drug delivery or as structure supports for tissue engineering.

In various aspects, CNTs are defunctionalized. In various embodiments, the CNTs are defunctionalized prior to complexing with the myocyte cell. The process of defunctionalizing CNTs may proceed according to any suitable method, including chemical treatment and heat. The CNT may be defunctionalized prior to or after deposition onto a substrate, or in the absence of substrate deposition. In various embodiments, defunctionalizing proceeds by subjecting the CNT to heat, at any suitable temperature. In various other aspects, the CNT is functionalized. The functional groups may be complexed for any suitable purpose, including, among others, facilitating additional modes of binding to a target myocyte cell.

In further aspects, a CNT-based composition is provided. The composition includes a plurality of carbon nanotubes complexed to one or more cardiac myocyte cells. The plurality of CNTs may contain single-wall carbon nanotubes, a double-wall carbon nanotubes, multi-wall carbon nanotubes, or mixtures thereof.

In further aspects, a method of forming a CNT complex is provided. The method includes the steps of obtaining a plurality of CNTs and dispersing and/or suspending the CNTs with an agent, such as a dispersing or suspension agent. In further embodiments, the dispersion may be deposited onto a substrate. Any suitable substrate may be used in accordance with the invention, including glass, polymers, or any other suitable material. In various embodiments, a glass substrate is used. Deposition proceeds until achieving a desired volume and/or thickness of the resulting layer, or "film." A population of myocytes, such as cardiac myocytes, may be deposited onto the substrate and complexed with the CNTs, resulting in a complex between the CNTs and myocyte cells.

In further aspects, a method of modulating the electrophysical properties of myocyte cells is provided. She aspect includes obtaining a plurality of CNTs and obtaining a population of myocyte cells, for example cardiac myocyte cells, and forming a CNT-myocyte complex as described further herein above and below. In various embodiments, the modulated electrophysical function of the myocyte is characterized by achieving a more negative resting potential compared to control and an increased AP firing compared to control. In various embodiments, these potentials are defined by ranges from about -30 mV to about -90 mV, from about -30 mV to about -75 mV, and from about -30 mV to about -60 mV.

In further aspects, a method of stimulating the proliferative capacity of myocyte cells is provided. The aspect includes obtaining a plurality of CNTs and obtaining a population of myocyte cells, for example cardiac myocyte cells, and forming a CNT-myocyte complex as described further herein above and below. The aspect further includes expanding the population of the myocyte cells under suitable conditions as further exemplified below.

In still further aspects, methods of treating subjects are provided, for example, by administering, implanting or otherwise contacting CNTs in accordance with the invention to treatment areas of interest, such as to regions or tissue-specific treatement areas of interest, and, for example, by otherwise modulating the electrophysical properties, proliferative capacities, and/or viability potentials of myocyte cells in accordance with the various aspects and embodiements of the invention; wherein such subjects suffer, or are predisposed to suffer from, arrhythmia, conduction disease, cardiomyopathy, heart failure, atherosclerosis, peripheral vascular disease, dyslipidemia, hyperbetalipoproteinemia, hypoalphalipoproteinemia, hypercholesterolemia, hypertriglyceridemia, familial-hypercholesterolemia, cardiovascular disorders, angina, ischemia, cardiac ischemia, stroke, myocardial infarction, reperfusion injury, angioplastic restenosis, hypertension, vascular complications of diabetes, bladder disease and conditions, and skeletal muscle diseases, including muscular dystrophies.

The present invention reveals, among others, the surprising discovery that myocytes modify their viability, growth, differentiation and electrophysiological properties when interacting with carbon nanotubes.

### EXAMPLES

In order that the invention may be better understood, the following examples are set forth. These examples are for purposes of illustration only, and are not to be construed as limiting the scope of the invention in any manner. In the following examples, as well as elsewhere in the specification and claims, temperatures are in degrees Celsius, and the pressure is atmospheric unless indicated otherwise.

Although the foregoing invention has been described in some detail by way of illustration and by example for purposes of clarity of understanding, it will be readily apparent to those skilled in the art in light of the teachings of this invention, that certain changes and modifications may be made thereto without departing from the scope of the appended claims.

### Example 1 - Synthesis of Multi-Walled Carbon Nanotubes

Multi-wall carbon nanotubes (MWCNT) of 20-30 nm, were purchased from Nanostructured & Amorphous Materials, Inc. (HiPCO nanotubes, Carbon Nanotechnology Inc., Houston, Texas), used as received, and prepared (functionalized via 1,3-dipolar cycloaddition reaction) as previously described in Lovat et al., Carbon nanotube substrates boost neuronal electrical signaling. Nano Lett. 5, 1107-1110, (2005). Thermogravimetric analysis (TGA) was performed using TGA Q500 from TA Instruments (New Castle, DE, US). The analysis was performed under N2 or under air, by equilibrating at 100°C, and following a ramp of 10 °C/min up to 1000°C. In the case of defunctionalized MWCNTs, the material was analyzed after being exposed to high temperature (350°C) under N2.

### Example 2 - Fabrication of Glass Substrate

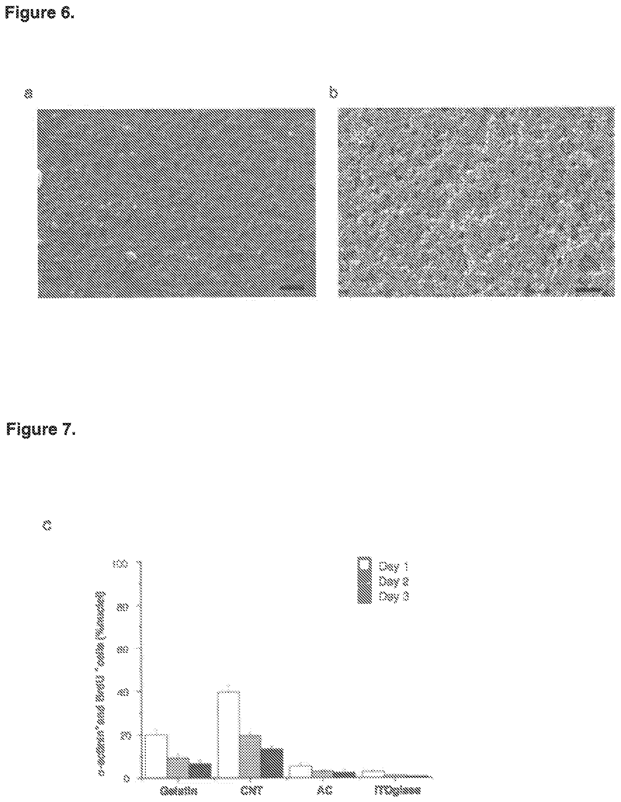

MWCNTs were dispersed in N,N'-dimethylformamide (DMF) at a concentration of 0.01 mg/mL and 0.2 mL. The dispersed solution of MWCNTs was then deposited up to 10 times onto a glass substrate to achieve a total volume of 2 mL. The density of the MWCNT film over the glass was about 7x10⁻⁵ mg/mm². The thickness of the film was measured by atomic force microscopy (AFM). A glass substrate comprising MWCNTs was obtained.
MWCNT film was characterized by sheet resistance measurements obtained using a Jandel four-tips probe. The thickness of the film was measured by atomic force microscopy (AFM) and the conductivity of the material was extracted. MWCNT-coated glass substrate AFM images were analyzed by Gwyddion SPM data analysis software and average roughness calculated as Rms was derived. AFM measurements were registered with a Veeco V in tapping mode. Phosphorus (n) doped Silicon tips with a resonance frequency of 273-325 kHz and a constant force of 20-80 N/m were used. The calculated value of conductivity for the MWCNT used in this study is equal to 3.82 x 105 S/cm. A SEM image of a sample of the MWCNT growth substrate used in these experiments is depicted in the FIGS. 6A and 6B.

Industrial quality Indium-Tin-Oxide (ITO) substrates were obtained by depositing a thin conductive layer of TIO on glass substrates. Because of its low electrical resistance (0.005-5 Ω mm), we employed ITO substrates as a cell culture smooth substrate and as an alternative to nanotubes (Cellot et al., Nanoneurosci, 1, 10-16 (2009)). Amorphous Carbon (AC) was purchased from Sigma-Aldrich (Carbon nanopowder ≥ 99%). The material was treated in the exact way as previously described for the functionalization of MWCNTs using AC as scaffold (Toma et al, Nat Chem, 2, 826-831 (2010)).

### Example 3 - Defunctionalizing the MWCNTs

The substrate complex was then treated for 20 minutes in an oven under N2 atmosphere to remove chemical impurities, thereby achieving a defunctionalized MWCNT.

### Example 4 - Culture of Neonatal Rat Ventricular Cardiac Myocytes

Neonatal Rat Ventricular Myocytes (NRVM) were prepared from six 1-3 days old pups according to an enzymatic digestion procedure. Briefly, ventricles were separated from the atria using scissors and then dissociated in CBFHH (calcium and bicarbonate-free Hanks with Hepes) buffer containing 500µg/ml of Collagenase type 2 and 1 mg/ml of Pancreatine. Cardiomyocytes were enriched (>90% purity) over non-myocyte cells by two subsequent pre-plating steps into 100-mm dishes in DME containing 10% horse serum, 5% bovine calf serum and 2 mg/ml vitamin B12. Unattached myocytes were collected and plated at a density of 2.0x105 per slide alternatively coated with 0.2% gelatin or CNT for immunofluorescence staining. After 24h, the culture medium was changed and cells were subjected to the different treatments and subsequent analyses.

Although NRVM are terminally differentiated cells, they preserve residual proliferation capacity in a neonatal rat heart for few days after birth. Additional proliferation capacity in adult cells is also contemplated and within the scope of the various aspects of the invention.

### Example 5 - Transmission and Scanning Electron Microscopy

Transmission electron microscopy (TEM) analyses were performed on a TEM Philips EM208 (Koninklijke, Philips Electronics N.V., Amsterdam, the Netherlands) using an accelerating voltage of 100 kV. About 1 mg of compound was dispersed in 1 mL of solvent. Then, one drop of this solution was deposited on a TEM grid (200 mesh, Nickel, carbon only). Scanning electron microscopy (SEM) measurements were carried out with a Zeiss Supra™ microscope.

For TEM analysis, NRVM were fixed after 3 days of culture in vitro with a solution containing 2.5% glutaraldehyde (Fluka, Italy) in 0.1 M cacodylate buffer, pH = 7.4, for 1 h at room temperature. Cultures were then washed in cacodylate buffer for several times and then transferred into a PBS solution containing 1% osmium tetroxide (Fluka) for 1 h at room temperature. Cultures were then dehydrated in graded ethanol and embedded in epoxy resin (DER 332-732). Planar and sagittal ultrathin sections (85 nm) were obtained by cutting the samples in orthogonal planes to the surface. Sections were then collected onto 300 mesh nickel or copper grids, stained with uranyl acetate and lead citrate (Fluka) and examined with a Philips EM208 by the digital acquisition system GATAN TVC 673. The morphological analysis was performed on digital images obtained from 50 randomly-selected grid-fields.

For scanning electron microscopy (SEM) measurements, the samples were sputter-coated with gold in an Edwards S150A apparatus (Edwards High Vacuum, Crawley, West Sussex, UK), and examined with a Leica Stereoscan 430i scanning electron microscope (Leica Cambridge Ltd., Cambridge, United Kingdom),

### Example 6 - Immunohistochemical staining

The primary antibodies used were as follows: mouse anti α-sarcomeric actinin (EA-53), 1:100 (STIGMA); rat monoclonal (BU1/75) anti-BrdU, 1:100 (AbCam), and rabbit polyclonal anti Phospho-Histone H3 (Ser28) 1:200 (Cell Signaling Technology). The secondary antibodies used were as follows: goat anti-mouse conjugated to Alexa Fluor 488; goat anti-rat conjugated to Alexa Fluor 555; goat anti-rabbit conjugated to Alexa Fluor 594; all at 1:1,000 (Invitrogen). Cells were fixed in PBS containing 3%PFA and 2%sucrose for 10 min; aldehydes were quenched with 0.1M Glycine in PBS for 15 min at room temperature. Cells were permeabilized with 1% Triton X-100 for 5 min, blocked in 2% BSA and 0.05% sodium azide in PBS (blocking buffer) for 1h, and incubated with primary antibodies from 2h to overnight. Alexa Fluor 488- or Alexa Fluor 555- and 594-conjugated secondary antibodies (Invitrogen) were incubated from 45min to 1h at room temperature. All washes were in PBS 0.2% Tween 20. Cell nuclei were stained with DAPI and samples were mounted in Vectashield (Vector Laboratories).

Images were acquired at room temperature with a DMLB upright fluorescence microscope (Leica) equipped with a charge-coupled device camera (CoolSNAP CF: Roper Scientific) using MetaView 4.6 quantitative analysis software (MDA Analytical Technologies). For image acquisition, the following objectives were used: HCX PL apocromatic 63x/1.32-0.6 NA, HCX PL Fluotar 40x/0.75 NA, HCX PL N-Plan 20x/0.40 NA, and HCX PL N-Plan 10x/0.25 NA (all from Leica).

### Example 7 - BrdU pulse labeling and detection

After pulse-labeling of NRVM (10 µM BrdU for 2h), staining was performed according to the manufacture's instructions (BD Bioscences). In brief, cultures were fixed in 3% PFA and 2% sucrose in PBS, pH 7.6; washed three times for 10 min with PBS 1% Triton X-100; and incubated with 1M HCL for 10 min on ice, 2M HCL for 20 min at 37°C. After DNA denaturation, cells were incubated with 0.1M sodium-borate buffer, pH 8.4 for 12 min room temperature, and then washed three times with PBS 1% Triton X-100, blocked with 2% BSA in PBS 1h at room temperature, and incubated with anti-BrdU antibody for 2h at 37°C. All subsequent steps were as described above in the "Immunohistochemical staining" section. Each slide was then analyzed by counting, in a blind fashion, the number of BrdU-positive and BrdU-negative cells over 10 high-magnification fields for each condition (gelatin and CNT substrates).

### Example 8 - Almar Blue Cell Viability reagent

At day 2, 1/10th volume of AlmarBlue* reagent (Molecular Probes, Invitrogen) was directly added to the cells medium and incubated for 1 to 36 hours at 37°C in a cell culture incubator, protected from direct light. Sensitivity of detection increases with longer incubation times. The resulting red absorbance was read at 570nm wavelength on a plate reader, and measurements were normalized to the 600 nm wavelength reference value. Larger absorbance emission intensity values correlate to an increase in total metabolic activity from cells in the well. All the measurements were performed in triplicate on 3 independent experiments.

At day 3, cardiomyocyte syncytia beating domain surface was determined for 20 randomly selected syncytia of approximately 7 cells per experiment, and averaged to provide an N value of one. Cells were examined by standard epifluorescence microscopy performed with a Zeiss LSM 510 META Confocal Microscope (Carl Zeiss Microscopy, Jena, Germany), using 40x objective. The area of the syncytia was determined using LSM 510 Image Examiner™ software. Grouped data is presented as mean ± standard (See, e.g., FIG. 2D),

### Example 9 - Electrophysiological recordings

Whole cell patch clamp recordings were obtained at 37°C, both in current and voltage clamp configurations, employing patch-pipettes (3.5-5 MΩ) under GΩ patch sealing, using a Multiclamp 700B (Axon Instruments, Foster City, CA,USA). Recording solution contained (mM): NaCl 140, KCl 5, MgCl2 1, CaCl2 2, HEPES 10, glucose10, pH 7.4, Patch pipettes contained (mM): 130 K-gluconate, 15 KCl, 5 NaCl, 5 Mg-ATP, 1 MgCl2, 5 EGTA, 1 CaCl2, and 10 HEPES, pH=7.2. Recordings were performed on NRVM after 2-3 days of in vitro differentiation. Single isolated NRVM, still not fused to form syncytia, were identified under visual investigation, by means of their ability of beating.

Capacitance and input resistance were measured in voltage clamp mode at -60 m V, with a subthreshold 100 ms duration hyperpolarizing voltage step (5 mV). Bridge balancing was continuously monitored and adjusted. Spontaneous activity was observed in current clamp configuration at the resting potential of single NRVMs, while induced action potentials (AP), obtained by injecting suprathreshold depolarizing current pulses (0.5 nA, 5 ms), were studied maintaining all cells at -70 mV resting potential (15 trials at 0.2 Hz for each cell). Current and voltage clamp responses were amplified, digitized at 10-20 kHz with the pCLAMP 10 software (Axon Instruments, Foster City, CA) and stored for further analysis.

### Example 10 - Statistical analysis

All electrophysiological values from batches of cultures subjected to the same experimental protocols were pooled together and expressed as mean ± standard error with n=number of cells. Statistical analysis was carried out using the Student's and Chi Square's tests (p<0.05).

### Example 11 - Morphology analysis of CNTs and cardiomyocytes

After functionalization, a CNT-DMF (N,N-dimethylformamide) solution was deposited onto glass coverslips and subsequently dried. Prior to cell seeding, carbon nanotube-coated glass coverslips were exposed to high temperature to remove the organic functionalization, producing pristine (not-functionalized) carbon nanotubes, for example, as further described in Examples 1 and 2. Defunctionalization in accordance with this invention yielded a low metal content, < 5%.

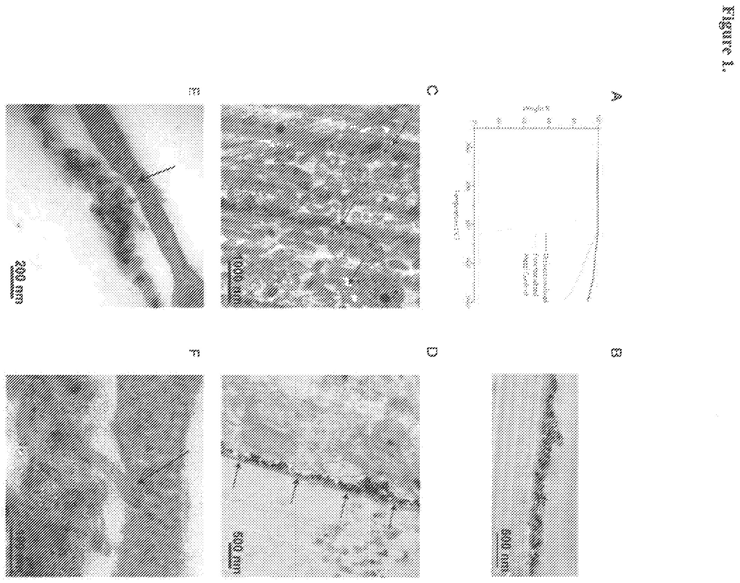

Thermogravimetric analysis (TGA) was performed to characterize CNTs and provide information about their degree of functionalization (under N2 atmosphere) and metal content (under air). FIG. **1A** shows the TGA curves showed a very low (<5%) MWCNT metal contentand degree of functionalization, and subsequent repristinization after the annealing procedure (Cellot et al., J Neurosci, 31, 12945-53(2011)). Neonatal rat ventricular myocytes (NRVMs) were cultured on the defunctionalized carbon-nanotube scaffolds. To examine the interactions between nanotube layers and cell membranes, the NRVM cultures were examined by transmission electron microscopy (TEM). FIG. **1B** shows a CNT layer deposited onto the glass substrates and characterized by TEM. The average thickness of the carbon nanotube layer was 162.75 ± 11.4 nm (n=42, measurements, randomly sampled from 3 different TEM images). The morphology and organization of NRVM grown on nanotubes was assessed by analyzing planar sections, as shown in FIG. **1C****.** These same samples were further analyzed in sagittal sections, to visualize the zone of nanotubes-membrane contact, as shown (arrows) in FIG. **1D****.** Higher mangnification of the samples reveal the nanotubes further, and a "pinching" of nanotubes on the cell membrane, as shown in FIGS. **1E** and **1F** represents a SEM image where a panoramic view of the specimen is given.
These ultrastructural interactions between nanotubes and NRVM membranes were observed in all sections explored and demonstrate intimate and discontinuous interactions of nanotubes with neuronal membrane surfaces. While not being bound by any particular theory, these interactions are believed responsible in part for inducing observed changes in membrane electrical behavior.

In accordance with the present invention, NRVM are grown on CNT substrate without displaying signs of toxicity. Moreover, many points of contact between cell membrane and CNTs are provided.

### Example 12 - Viability and replication analysis

In order to evaluate the effect of CNTs on cell viability and replication, the metabolic activity of NRVM cultures was compared on CNT substrate and on gelatin using an AlmarBlue-based colorimetric assay (FIG. **2A****).** Although virtually no differences were detected between the two substrates within the first 18 h, the measured relative absorbance was 2.5 times higher with cells plated on CNTs when compared to the gelatin ones after 36h of culture, and this difference was statistically significant (n=6, p<0.05). FiG. **2A****.** The histogram shows mean ± sem, of total number of α-actinin positive cells from at least three independent experiments, P<0.05. It was surprisingly found that the CNTs and gelatin substrates exert different metabolic and proliferation properties on neonatal myocytes.

In a second set of experiments, the proliferating capacity of NRVM was investigated. NRVMs were plated on the two different substrates, gelatin and CNTs. To this purpose, cells were analyzed in complete medium at three different time points in culture; specifically on post-plating day 1, 2, and 3. Subsequently, cells were fixed and immunostained for α-sarcomeric actinin to distinguish α-actinin-positive cardiac myocytes from from α-actinin-negative fibroblasts. Nuclei of all cells were stained with DAPI (FIG. **2B**).

The number of α-actinin-positive and negative cells were quantified in cultures at the different time points. After 24 h from plating (day 1), neonatal cardiomyocytes (NRVM) were detected as small round cells expressing poorly organized α-actinin. At day 2, they appear increased in size and shown progressively more organized sarcomeres. At day 3, they appear larger and differentiated. Nuclei were stained with DAPI. Top row depicts gelatin substrate and bottom row depicts CNT substrate. The fluorescence is represented in the two channels. Bars: 50 µm. FIG. **2B****.**

On day 3, both NRVM and fibroblasts have increased their number with respect to day 1. In particular, NRVM increased 2 times on CNT substrate while, on gelatin substrate only 1.5 times. Although the ratio between NRVM/fibroblasts remained constant (6:1) on days 1-3 for the two substrates, the number of cardiomyocytes was found to be doubled on CNT compared to the gelatin substrate. Quantification of total myocardial cells from newborn rats on gelatin and CNT substrates at different time points in culture is depicted in the histogram in FIG. **2C**. The histogram show mean ±SEM, of total number of α-actinin-positive cells from at least three independent experiments, P<0.05.

Although NRVMs are terminally differentiated cells, they are known to maintain a modest amount of residual proliferative capacity both following birth in vivo and when placed in cell culture. In the present studies, although the plating efficiency of NRVMs was slightly lower on MWCNT with respect to gelatin (66% versus 76.5%, respectively), the proliferative capacity of cardiomyocytes on carbon nanotubes was significantly higher. Specifically, this substrate was able to sustain a doubling in cell number after 3 days.

As indicated in FIG. **2C** and Table 1, this difference in proliferative capacity was statistically significant (n≥3 independent experiments, P=0.030) (Table I and FIG. **2C**). Proliferation of contaminating cardiac fibroblasts was unaffected by plating on carbon nanotubes compared to gelatin (FIGS. **2D** and **2E**). These results demonstrate that carbon nanotubes can actively and selectively influence the proliferating capability of NRVM, without similar effects on fibroblasts activity. Furthermore, on day 3 the size of the syncytia-beating domains was significantly increased (*P*=0.0184, FIG. **2F****).**

**Table 1. Quantification of cell proliferation on CNT and gelatin substrates.**

| **Cell type** | **Substrate** | **Day 1** | **Day 2** | **Day 3** |
|---|---|---|---|---|
| **CM** | **Gelatin** | 1.8x10⁵±1.5x10⁴ | 2.1x10⁵±1.3x10⁴ | 2.6x10⁵±1.3x10⁴ |
| | **CNT** | 2.5x10⁵±2.4x10⁴ | 4.2x10⁵±2.1x10⁴ | 4.9x10⁵±2.6x10⁴ |
| **Fibroblasts** | **Gelatin** | 3x10⁴±2.1x10³ | 3.5x10⁴±2.3x10³ | 4.1x10⁴±2.3x10³ |
| | **CNT** | 4x10⁴±4x10³ | 6.5x10⁴±3.3x10³ | 8.1x10⁴±4.2x10³ |

This observation raised two intriguing possibilities: either different substrates influence cell viability or different substrates influence the proliferative potential of immature cardiac myocytes.

To test this hypothesis, we analyzed the number of cycling cells present in each culture group (gelatin and CNT) in a time course experiment during three days of differentiation. For this purpose, cells cultured in complete medium were pulsed for 2 hours with 10mM BrdU before fixation. Then, the number of cells that had incorporated the nucleotide analogue was assessed by immunofluorescence, using anti BrdU specific antibodies.

After 24 h from plating, NRVM appeared as small and rounded in shape and with poorly organized α-actinin both on gelatin and CNT substrates. Over the next 72 hours, NRVM progressed and differentiated, and increased in size, as well as showing progressively more organized sarcomeres, fused in multinucleated aggregates, also referred to as syncytia. From day 1, immature cardiomyocytes started beating on both the substrates.

BrdU and phosphorylated-histone H3 incorporation in cardiac myocytes on different substrates was assessed, as shows generally in FIG. **3A****.** At day 2 twice as many BrdU-positive cardiomyocytes were observed on CNT substrate compared to gelatin substrate. The cells were positive for BrdU incorporation at all time points (FIG. **3A****, left panel** for representative images). The histogram shows mean ± sem, of total number of BrdU-positive cells plated on gelatin and CNT substrates from at least three independent experiments, P=0.032; Bar=50µm (FIG. **3A****, right panel),** Quantification revealed that ∼39% of nuclei in NRVM at day 1 scored positive for BrdU incorporation on CNT substrates, whereas only ∼20% of nuclei were found positive for BrdU on gelatin substrates. The percentage of BrdU positivity progressively decreased to values corresponding to ∼22% and ∼9% at day 2, and to ∼13% and 6,5% at day 3 on CNT and gelatin substrate, respectively. The differences in cell number on the two different substrates were statistically significant (n=6, p<0.010). At each time point examined there were twice as many dividing cells on carbon nanotubes than on the gelatin substrate.

To confirm these data, NRVM were immunostained at day 1 with an antibody against phosphorylated-histone H3, a marker of cell division that is only expressed in proliferating cells. Representative images of positivity to phosphorylated-Histone-H3 (Ser28) immunostaining is provided (FIG. **3B** **left panel** for representative images, gelatin (top)- and CNT (bottom)-substrates). Nuclei were stained with DAPL Approximately 28% of NRVMS nuclei stained positive for the antibody on CNT substrate, while only ∼19% were positive on gelatin (FIG. **3B**). The differences in positive proliferating cell number on the two substrates were not statistically significant.

To test the specificity of MWCNT proliferating ability, the effects of different materials used as NRVM growth platforms were compared. NRVM were cultured on indium tin oxide (ITO)-glass, which characterized by high electrical conductivity gelatin coated ITO-glass (Cellot, G. et al., Nat. Nanotechnol. 4, 126-33 (2009)), used as control, and amorphous carbon coated glass, representing a control for the carbon substrate (FIGS. **6A** and **6B**). Cardiac cells were stained with BrdU and examined at 24, 48 and 72 hours. On amorphous planar carbon surface, BrdU positive NRVMs were significantly less (day 1: 5.43%, day 2: 2.91%, day 3: 2.17%) than on carbon nanotubes, while fibroblast growth seems to be favored (day 1: 2.17%, day2: 7.76%, day 3: 5.88%). The same effect was seen on ITO-glass surface (positive BrdU NRVM day 1: 2.73%, day 2: 1.08%, day 3: 0.51%; fibroblasts day 1: 4.37%, day 2: 6.03%, day 3: 7.3%) while gelatin coated ITO-glass was comparable to the gelatin coated control glass (day 3: 6.02%). The profile of proliferation of BrdU positive NRVM grown on carbon nanotubes, gelatin, amorphous carbon and ITO-glass at day 1, 2 and 3, was compared (FIG. 7); results of which indicate that carbon nanotubes exert a unique and selective proliferative effect on cardiomyocytes.

In accordance with the present invention, the results demonstrate the surprising findings that NRVM maintain viability and significantly increase proliferative capacity when stimulated by the CNT substrate.

### Example 13 - Analysis of resting potential and spontaneous action potential generation in cardiomyocytes

Electrophysiological evaluations were conducted on single isolated cardiac myocytes grown on MWCNTs or on gelatin control substrates. Cells were recorded after 2-3 days of differentiation *in vitro* in whole cell patch clamp configuration. First measured and quantified were membrane passive properties, such as capacitance, input resistance and resting potential. These parameters are widely accepted indicators of the degree of cellular development and health.

Both gelatin and carbon nanotube cultures showed similar input resistance. FIG. **4A****.** NRVM grown on carbon nanotube substrates displayed values of resting potential 24% more negative than those of gelatin NRVM (gelatin NRVM: -38±2 mV, n=72 cells and CNT NRVM: -47±2 mV, n=65 cells,, p<0.05). FIG. **4B****.** However, no differences were found in capacitance and input resistance between the two groups (19±1 pF and 24±2 pF; 1±0,9 GΩ and 1±0,9 GΩ; n=72 and n=65, gelatin and MWCNTs, respectively).

The active electrical properties of NRVM developed on both culture groups was also examined (gelatin and MWCNTs). The ability of NRVM to spontaneously generate action potentials (APs) was monitored under current clamp configuration. In both conditions of growth, single NRVM were able to generate spontaneous APs only when their resting potential was more negative than -35 mV. Thus, the probability (expressed as %) of NRVM able to fire APs was significantly higher in the presence of MWCNTs when compared to gelatin sister cultures (64±3.5% and 46.6±4.5%; n=70 and n=66 cells; MWCNTs and gelatin, respectively. FIG. **4C****.** Spontaneously firing NRVM, regardless of their culturing conditions, generated APs with comparable frequencies (0.78±0.12 Hz and 0.81±0.3 Hz; n=13 and n=9, gelatin and MWCNTs, respectively. FIG. **4E****.**

In a separate set of experiments we compared evoked APs in NRVM of both growth conditions. NRVM were maintained in current clamp configuration at -70 mV, and APs were induced by injecting brief (5 ms) square depolarizing current steps (0.5 nA; 0.2 Hz).

Superimposed traces of evoked APs were sampled from a gelatin and a CNT NRVM (gray traces; black trace represents the average of 15 trials). FIG. **4F****.** In both cases, the recordings show AP shape and kinetics similar to those usually described for NRVM (Nerbonne, J. M.; Kass, R, S. Physiol Rev, 85, 1205-53 (2005).

APs features were measured and quantified. Table 2. The data illustrates a similarity of APs between the two culturing conditions, with the exception of a tendency (although not significant) to display shorter APs in CNT cultures.

**Table 2. Action Potential (AP) features of NRVM on CNT substrate or gelatin (control).**

| | **Gelatin (n=38 cells)** | **CNT (n=35 cells)** |
|---|---|---|
| Rise slope (mV/ms) | 52±3 | 55±2 |
| Peak amplitude (mV) | 145±3 | 137±3 |
| AP duration (50% reρolarization) (ms) | 82±16 | 62±10 |
| AP duration (90% repolarization) (m) | 166±26 | 130±17 |
| Max afterhyperpolarization (mV) | -1.04±0.3 | -1.22±0.3 |

### Example 14 - Skeletal Muscle Growth and Maturation Analysis

The ability of CNT scaffolds to enhance growth and electrical properties of skeletal myocytes was examined.

### A. Cell culture

Mouse C2C12 skeletal muscle cells (Blau et al. Cell. Apr;32(4):1171-80 (1983)) derived from C2 cells (Yaffe and Ora, Nature 270, 5639: 725-727 (1977)). The proliferating myoblasts were maintained in Dulbecco's modified Eagle's medium (DMEM) supplemented with 10% fetal bovine serum (FBS) (growth medium).

Cells were collected and plated at density 1x10⁵ per slide and alternatively coated with 0.2% gelatin or carbon nanotube for immunofluorescence staining. After 24h and 48h, the culture medium was changed and cells were subjected to the different treatments and subsequent analyses.

### B. Immunocytochemical staining

Immunocytochemical staining was conducted. The primary antibodies used were as follow: mouse anti actin (SC-8432), 1:50 (Santa Cruz Biotechnology, Santa Cruz, CA). The secondary antibodies used were as follows: goat anti-mouse conjugated to Alexa Fluor 488, 1:1,000 (Invitrogen, Grand Island, NY). Cells were fixed in PBS containing 3%PFA and 2% sucrose for 15 min; aldehydes were quenched with 0.1 M Glycine in PBS for 15 min at room temperature. Cells were permeabilized with 1% Triton X-100 for 5 min, blocked in 2% BSA and 0.05% sodium azide in PBS (blocking buffer) for 1 h, and incubated with primary antibodies for 2h at RT. Secondary antibody was incubated from 45min to 1h at room temperature. All washes were in PBS 0.2% Tween 20. Cell nuclei were stained with Propidium
loide PI (3µg/ml final concentration) and samples were mounted in Vectashield (Vector Laboratories, Burlingame, CA). Images were acquired at room temperature with a DMLB upright fluorescence microscope (Leica Cambridge Ltd., Cambridge, United Kingdom) equipped with a charge-coupled device camera (CoolSNAP CF: Roper Scientific, Ottobrunn Germany) using MetaView 4.6 quantitative analysis software (MDS Analytical Technologies, Toronto, ON, Canada). For image acquisition, the following objectives were used: HCX PL apochromatic 63x/1.32-0.6 NA, HCX PL Fluotar 40x/0.75 NA, HCX PL N-Plan 20x/0.40 NA, and HCX PL N-Plan 10x/0.25 NA (all from Leica).

### C. C2C12 cell proliferation assay

Cultures were fixed in 3% PFA and 2% sucrose in PBS, pH 7.6, washed three times for 10 min with PBS 1% Triton X-100, blocked with 2% BSA in PBS 1h at room temperature, and then treated with DAPI to stain the nuclei. Each slide was then analyzed by counting, the number of blue nuclei over 10 high-magnification fields for each condition (gelatin and carbon nanotube substrates). All the measurements were performed in triplicate from 3 independent experiments.

### D. Results

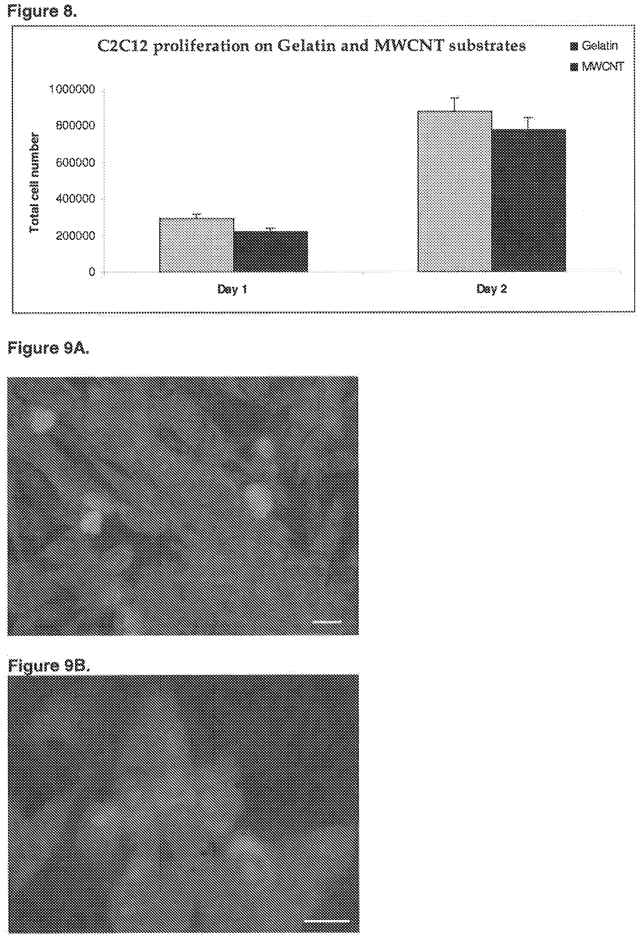

Carbon nanotube and gelatin substrates induce different proliferation behaviors in cultured C2C12 myobalsts. DAPI assay was used as indicator of cell proliferation (FIG. 8). Quantification of total numbers of C2C12 detected on gelatin and on MWCNT substrates at the different days in culture is shown in the histogram (mean ± standard error of total number of nuclei). On day 2, as expected, C2C12 cells increase in number on gelatin, since C2C12 cells are characterized by active proliferation. The rate of growth was comparable on MWCNT (P=NS), showing that carbon nanotubes do not interfere with the high proliferative capacity of C2C12.

Representative images of C2C12 stained for actin cytoskeleton and Propidium Ioide for nuclei were obtained (FIG. 9A). On MWCNT the skeletal myocytes show a more organized structure with some multinucleated fibers, and pseudo-myotubes (FiG. 9B). These structures are not evident on gelatin substrates, indicating that the tested MWCNT can promote phenotype maturation.

### Example 15 - Electrophysiologica Recordings

Electrophysical recordings of C2C12 myoblasts were assessed. Cell activity was monitored by means of whole cell patch clamp recordings after 12 hours from the seeding of cells on the substrates (gelatin and MWCNT). Membrane passive properties were assessed (Table 3).

**Table 3. Electophysiological Recordings of C2C12 cells:**

| | Capacitance (pF) | Input resistance (MΩ) | Resting potential (mV) | |
|---|---|---|---|---|
| Gelatin | 29±2 | 98±9 | -61±4 | n=6 cells |
| CNT | 63±4 | 86±35 | -59±2 | n=6 cells |

| | | | | |
|---|---|---|---|---|
| The data are expressed as mean±s.e.m. | | | | |

The cells grown on MWCNT show a higher value of capacitance in comparison with control, while other parameters appear similar between the two groups.

Examples 14 and 15 demonstrates that that the beneficial effect shown by carbon nanotubes on cell growth and maturation extends to all striated muscle, to include cardiac myocytes and skeletal myocytes, CNTs treatment in accordance with the present invention promotes active proliferation and phenotype maturation compared to control.

In the various examples, and without being bound by any particular theory, the inventors found that carbon nanotubes develop close contacts with the NRVM membrane and penetrate into the cell. This interaction did not damage the cell, but surprisingly was found to enhance cell viability and proliferation potential. The carbon nanotube scaffolds were also found to induce a more negative resting potential compared to control and an increased AP firing. In neonatal cardiac myocytes, the resting potential becomes progressively negative as the cells develop into the adult phenotype, leading to a further inventive aspect of increasing and/or promoting cell maturation.

In accordance with the present invention, carbon nanotubes can modify viability, proliferation, growth, maturation and electrophysiological properties of striated muscle cells, such as cardiac myocytes and skeletal myocytes. The subject carbon nanotubes prolong the proliferative state of myocutes, which maintains some cells in an undifferentiated state, and accelerate the maturation of the differentiated myocytes.

Numerous characteristics and advantages have been set forth in the foregoing description, together with details of structure and function. The disclosure, however, is illustrative only, and changes may be made in detail, especially in matters of shape, size, and arrangement of features, within the principle of the invention, to the full extent indicated by the broad general meaning of the terms in which the general claims are expressed.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein, and every number between the end points. For example, a stated range of "1 to 10" should be considered to 5 include any and all subranges between (and inclusive of) the minimum value of 1 and the maximum value of 10; that is, all subranges beginning with a minimum value of 1 or more, e.g. 1 to 6.1, and ending with a maximum value of 10 or less, e.g., 5.5 to 10, as well as all ranges beginning and ending within the end points, e.g. 2 to 9, 3 to 8, 3 to 9, 4 to 7, and finally to each number 1, 2, 3, 4, 5, 6, 7, 8, 9 and 10 contained within the range. It is further noted that, as used in this specification, the singular forms "a," "an," and "the" include plural references unless expressly and unequivocally limited to one referent.

## Claims

1. A carbon nanotube-based composition, comprising:
a plurality of carbon nanotubes complexed to one or more primary, cardiac myocyte cells selected from cardiac tissue.

2. The carbon nanotube-based composition of claim 1, wherein the plurality of carbon nanotubes is selected from a plurality of single-wall carbon nanotubes, double-wall carbon nanotubes, multi-wall carbon nanotubes, or mixtures thereof.

3. A method of forming a carbon nanotube complex, comprising the steps of:
a) obtaining a plurality of carbon nanotubes;
b) dispersing said carbon nanotubes with a dispersing agent wherein said dispersing agent is selected from any inorganic or organic solvent;
c) dispensing said dispersed carbon nanotubes onto a substrate, wherein dispensing said carbon nanotube onto the substrate yields a carbon nanotube film overlaying the substrate, and wherein said film has a density of 7×10⁻⁴ mg/mm² to 7×10⁻⁶ mg/mm2 ;
d) dispersing a population of previously obtained primary cardiac myocyte cells selected from cardiac tissue onto said substrate; and
e) complexing said carbon nanotubes with said cardiac myocyte cells, where said carbon nanotubes and said cardiac myocyte cells form a carbon nanotube complex over 24 hours.

4. The method of claim 3, wherein the dispersing agent is selected from ethanol, saline and N,N',dimethylformammide (DMF);

5. The method of claim 3, wherein said substrate comprises a glass substrate.

6. A method of improving the electrophysical properties of primary cardiac myocyte cells selected from cardiac tissue, comprising
a) obtaining a plurality of carbon nanotubes;
and,
b) complexing a previously obtained population of primary cardiac myocyte cells selected from cardiac tissue with said carbon nanotubes.

7. The method of claim 6 wherein the improved electrophysical property of said primary cardiac myocyte cell in complex with a carbon nanotube, recorded after 2 days of differentiation in vitro by whole cell patch clamp configuration, is **characterized by** at least one of:
inducing a 24% more negative resting potential when compared to the resting potential of control primary cardiac myocytes in gelatin culture which are not complexed to carbon nanotubes, or
an 18 - 60% increased probability of action potential firing when compared to the probability of action potential firing of said control primary cardiac myocytes in gelatin culture which are not complexed to carbon nanotubes.

8. The method of claim 3, wherein proliferative capacity of said primary cardiac myocyte cells is stimulated.

9. The method of claim 3 or claim 7 wherein the carbon nanotube is selected from a single-wall carbon nanotube, a double-wall carbon nanotube, and a multi-wall carbon nanotube.

10. The method of claim 9 wherein the carbon nanotube is a multi-walled carbon nanotube, and wherein said multi-walled carbon nanotube is defunctionalized prior to said step of complexing said primary cardiac myocyte with the carbon nanotube.

11. Carbon nanotube primary cardiac tissue selected cardiac myocyte complexes for use in the treatment of the following diseases: arrhythmia, conduction disease, cardiomyopathy, heart failure, atherosclerosis, peripheral vascular disease, dyslipidemia, hyperbetaliproteinemia, hypoalphalipoproteinemia, hypercholesterolemia, hypertriglyceridemia, familial-hypercholesterolemia, cardiovascular disorders, angina, ischemia, cardiac ischemia, stroke, myocardial infarction, reperfusion injury, angioplastic restenosis, hypertension, and vascular complications of diabetes, bladder disease and conditions, skeletal muscle disease, and muscular dystrophies.

## Patentansprüche

1. Kohlenstoffnanoröhrchenbasierte Zusammensetzung, umfassend:
eine Vielzahl von Kohlenstoffnanoröhrchen, die mit einer oder mehreren aus Herzgewebe ausgewählten primären Herzmuskelzellen komplexiert sind.

2. Kohlenstoffnanoröhrchenbasierte Zusammensetzung nach Anspruch 1, wobei die Vielzahl von Kohlenstoffnanoröhrchen aus einer Vielzahl von einwandigen Kohlenstoffnanoröhrchen, doppelwandigen Kohlenstoffnanoröhrchen, mehrwandigen Kohlenstoffnanoröhrchen oder Mischungen davon ausgewählt ist.

3. Verfahren zur Bildung eines Kohlenstoffnanoröhrchenkomplexes, folgende Schritte umfassend:
a) Erhalten einer Vielzahl von Kohlenstoffnanoröhrchen;
b) Dispergieren der Kohlenstoffnanoröhrchen mit einem Dispergiermittel, wobei das Dispergiermittel ausgewählt ist aus einem beliebigen anorganischen oder organischen Lösungsmittel;
c) Auftragen der dispergierten Kohlenstoffnanoröhrchen auf ein Substrat, wobei das Auftragen der Kohlenstoffnanoröhrchen auf das Substrat einen Kohlenstoffnanoröhrchenfilm auf dem Substrat erzeugt, und wobei der Film eine Dichte von 7x10⁻⁴ mg/mm² bis 7x10⁻⁶ mg/mm² aufweist;
d) Dispergieren einer Population zuvor erhaltener, aus Herzgewebe ausgewählter, primärer Herzmuskelzellen auf das Substrat; und
e) Komplexieren der Kohlenstoffnanoröhrchen mit den Herzmuskelzellen, wobei die Kohlenstoffnanoröhrchen und die Herzmuskelzellen in 24 Stunden einen Kohlenstoffnanoröhrchenkomplex bilden.

4. Verfahren nach Anspruch 3, wobei das Dispergiermittel ausgewählt ist aus Ethanol, isotonischer Kochsalzlösung und N,N'-Dimethylformamid (DMF).

5. Verfahren nach Anspruch 3, wobei das Substrat ein Glassubstrat umfasst.

6. Verfahren zur Verbesserung der elektrophysikalischen Eigenschaften von aus Herzgewebe ausgewählten primären Herzmuskelzellen, umfassend
a) Erhalten einer Vielzahl von Kohlenstoffnanoröhrchen;
und
b) Komplexieren einer zuvor erhaltenen Population von aus Herzgewebe ausgewählten primären Herzmuskelzellen mit Kohlenstoffnanoröhrchen.

7. Verfahren nach Anspruch 6, wobei die verbesserte elektrophysikalische Eigenschaft der primären Herzmuskelzelle in Komplex mit einem Kohlenstoffnanoröhrchen, aufgezeichnet nach 2 Tagen In-vitro-Differenzierung durch Whole-Cell-Patch-Clamp-Konfiguration, **gekennzeichnet ist durch** mindestens eins von:
Induzieren eines um 24 % negativeren Ruhepotentials im Vergleich zu dem Ruhepotential der Kontrollgruppe der primären Herzmuskelzellen in einer Gelatinekultur, die nicht mit Kohlenstoffnanoröhrchen komplexiert sind, oder
eine um 18-60 % erhöhte Wahrscheinlichkeit von Aktionspotentialfeuer im Vergleich zur Wahrscheinlichkeit von Aktionspotentialfeuer der Kontrollgruppe der primären Herzmuskelzellen in einer Gelatinekultur, die nicht mit Kohlenstoffnanoröhrchen komplexiert sind.

8. Verfahren nach Anspruch 3, wobei die Proliferationskapazität der primären Herzmuskelzellen stimuliert wird.

9. Verfahren nach Anspruch 3 oder Anspruch 7, wobei das Kohlenstoffnanoröhrchen ausgewählt ist aus einem einwandigen Kohlenstoffnanoröhrchen, einem doppelwandigen Kohlenstoffnanoröhrchen und einem mehrwandigen Kohlenstoffnanoröhrchen.

10. Verfahren nach Anspruch 9, wobei das Kohlenstoffnanoröhrchen ein mehrwandiges Kohlenstoffnanoröhrchen ist, und wobei das mehrwandige Kohlenstoffnanoröhrchen vor dem Schritt des Komplexierens der primären Herzmuskelzelle mit dem Kohlenstoffnanoröhrchen defunktionalisiert wird.

11. Komplexe aus Kohlenstoffnanoröhrchen und aus Herzgewebe ausgewählten primären Herzmuskelzellen zur Verwendung in der Behandlung folgender Erkrankungen: Herzrhythmusstörung, Erregungsleitungsstörung, Kardiomyopathie, Herzversagen, Atherosklerose, periphere Gefäßkrankheit, Dyslipidämie, Hyperbetalipoproteinämie, Hypoalphalipoproteinämie, Hypercholesterinämie, Hypertriglyceridämie, familiäre Hypercholesterinämie, Herz-Kreislauf-Erkrankungen, Angina, Ischämie, Herzischämie, Schlaganfall, Myokardinfarkt, Reperfusionsschaden, angioplastische Restenose, Bluthochdruck und vaskuläre Komplikationen von Diabetes, Blasenerkrankungen und - störungen, Skelettmuskelerkrankungen und Muskeldystrophien.

## Revendications

1. Composition à base de nanotubes de carbone, comprenant :
une pluralité de nanotubes de carbone complexés à une ou plusieurs cellules myocytes cardiaques primaires sélectionnées parmi du tissu cardiaque.

2. Composition à base de nanotubes de carbone selon la revendication 1, dans laquelle la pluralité de nanotubes de carbone est sélectionnée parmi une pluralité de nanotubes de carbone à paroi unique, de nanotubes de carbone à paroi double, de nanotubes de carbone à parois multiples, ou des mélanges de ceux-ci.

3. Procédé de formation d'un complexe de nanotubes de carbone, comprenant les étapes consistant à :
a) obtenir une pluralité de nanotubes de carbone ;
b) disperser lesdits nanotubes de carbone avec un agent de dispersion dans lequel ledit agent de dispersion est sélectionné parmi n'importe quel solvant inorganique ou organique ;
c) distribuer lesdits nanotubes de carbone dispersés sur un substrat, dans lequel la distribution dudit nanotube de carbone sur le substrat produit un film à nanotubes de carbone recouvrant le substrat, et dans lequel ledit film présente une densité de 7x10⁻⁴ mg/mm² à 7x10⁻⁶ mg/mm² ;
d) distribuer une population de cellules myocytes cardiaques primaires préalablement obtenues sélectionnées parmi du tissu cardiaque sur ledit substrat ; et
e) complexer lesdits nanotubes de carbone avec lesdites cellules myocytes cardiaques, où lesdits nanotubes de carbone et lesdites cellules myocytes cardiaques forment un complexe de nanotubes de carbone sur plus de 24 heures.

4. Procédé selon la revendication 3, dans lequel l'agent de distribution est sélectionné parmi l'éthanol, une solution saline et du N,N',diméthylformamide (DMF).

5. Procédé selon la revendication 3, dans lequel ledit substrat comprend un substrat en verre.

6. Procédé d'amélioration des propriétés électrophysiques de cellules myocytes cardiaques primaires sélectionnées parmi du tissu cardiaque, comprenant
a) l'obtention d'une pluralité de nanotubes de carbone ; et,
b) le complexage d'une population préalablement obtenue de cellules myocytes cardiaques primaires sélectionnées parmi du tissu cardiaque avec lesdits nanotubes de carbone.

7. Procédé selon la revendication 6, dans lequel la propriété électrophysique améliorée de ladite cellule myocyte cardiaque primaire en complexe avec un nanotube de carbone, enregistrée après 2 jours de différenciation in vitro par configuration patch clamp à cellule entière, est **caractérisée par** au moins une parmi : la provocation d'un potentiel de repos de 24 % plus négatif par rapport au potentiel de repos de myocytes cardiaques primaires de contrôle en culture de gélatine qui ne sont pas complexés à des nanotubes de carbone, ou une augmentation de 18-60 % de probabilité de déclenchement de potentiel d'action par rapport à la probabilité de déclenchement de potentiel d'action desdits myocytes cardiaques primaires de contrôle en culture de gélatine qui ne sont pas complexés à des nanotubes de carbone.

8. Procédé selon la revendication 3, dans lequel la capacité de prolifération desdites cellules myocytes cardiaques primaires est stimulée.

9. Procédé selon la revendication 3 ou la revendication 7, dans lequel le nanotube de carbone est sélectionné parmi un nanotube de carbone à paroi unique, un nanotube de carbone à paroi double et un nanotube de carbone à parois multiples.

10. Procédé selon la revendication 9, dans lequel le nanotube de carbone est un nanotube de carbone à parois multiples, et dans lequel ledit nanotube de carbone à parois multiples est défonctionnalisé avant ladite étape de complexage dudit myocyte cardiaque primaire avec le nanotube de carbone.

11. Complexes de myocytes cardiaques sélectionnés parmi du tissu cardiaque primaire à nanotubes de carbone à utiliser pour le traitement des maladies suivantes : arythmie, maladie de transmission, cardiomyopathie, insuffisance cardiaque, athérosclérose, maladie vasculaire périphérique, dyslipidémie, hyperbêtaliprotéinémie, hypoalphalipoprotéinémie, hypercholestérolémie, hypertriglycéridémie, hypercholestérolémie familiale, maladies cardiovasculaires, angine, ischémie, ischémie cardiaque, attaque, infarctus du myocarde, lésion de reperfusion, resténose angioplastique, hypertension et complications vasculaires de diabètes, troubles et maladie de la vessie, maladie des muscles squelettiques, dystrophies musculaires.
